Europäisches Patentamt
European Patent Office
Office européen des brevets

(11) Publication number: 0 278 089 A2

# EUROPEAN PATENT APPLICATION

(21) Application number: 87118508.8

(22) Date of filing: 14.12.87

(51) Int. Cl.[4]: C07D 207/16 , C07D 211/60 , C07D 275/02 , A61K 31/40 , A61K 31/445 , A61K 31/425

Claims for the following Contracting State: ES.

(30) Priority: 06.02.87 US 11981

(43) Date of publication of application: 17.08.88 Bulletin 88/33

(84) Designated Contracting States: AT BE CH DE ES FR GB IT LI LU NL SE

(71) Applicant: PENNWALT CORPORATION
Pennwalt Building Three Parkway
Philadelphia Pennsylvania 19102(US)

(72) Inventor: Griffith, Ronald C.
41 Northfield Gate
Pittsford New York 14534(US)

(74) Representative: Kraus, Walter, Dr. et al
Patentanwälte Kraus, Weisert & Partner
Thomas-Wimmer-Ring 15
D-8000 München 22(DE)

(54) **2-Azacyclocarboxamide derivatives.**

(57) Compounds are provided of the following general structure:

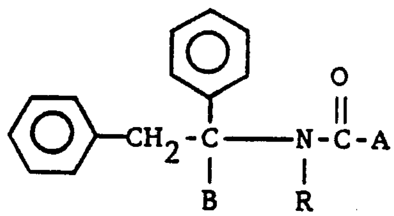

wherein A is 2-pyrrolidinyl, 2-piperidinyl or 4-thiazolidinyl and R and B are independently selected from hydrogen and methyl. They are useful for providing sedative and antiepileptic activity.

EP 0 278 089 A2

# 2-AZACYCLOCARBOXAMIDE DERIVATIVES

## Summary of the Invention

Novel substituted 2-azacyclocarboxamide derivatives have been prepared and found to possess useful sedative and especially antiepileptic activity.

## General Description

This invention relates to novel 2-azacyclocarboxamide compounds of the following general structure (1):

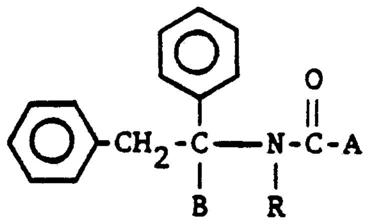

(1)

wherein A is 2-pyrrolidinyl, 2-piperidinyl or 4-thiazolidinyl and R and B are independently selected from hydrogen or methyl.

This invention also relates to diastereomeric and optically resolved forms and mixtures thereof, and to pharmaceutically acceptable acid addition salts of the compounds of general formula (1).

Compounds of this invention possess useful pharmaceutical properties. In particular they possess sedative and antiepileptic properties.

## Detailed Description

The 2-azacyclocarboxamides of general formula (1) as described fully above are conveniently prepared by suitable amide bond forming reactions from the corresponding amine intermediates of general formula (2):

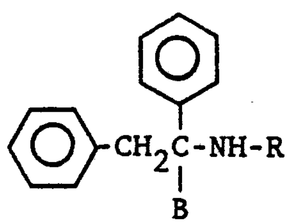

(2)

where B and R are as defined above.

Most of the amines of the general formula (2) are known compounds and may be purchased commercially or conveniently prepared by suitable modifications of the reported procedures. Amines of the general formula (2) which are not known are prepared by similar procedures. The preparation of amines of general formula (2) is described in the "Preparation of Intermediates" section.

The preferred method of amide bond formation consists of direct coupling of an amine of general formula (2) with a suitably N-protected cyclic amino acid, where the nitrogen is protected as a urethane, preferably as a benzyloxycarbonyl (CBZ) or a t-butyloxycarbonyl (BOC) urethane, in an inert solvent in the presence of a coupling reagent such as dicyclohexylcarbodiimide with or without 1-hydroxybenzotriazole or

other additives to provide N-protected coupled products. The protecting groups are then readily removed by either catalytic hydrogenation for the CBZ group or treatment with an acid such as trifluoroacetic or hydrochloric acid for the BOC group to provide compounds of the general formula (1).

The compounds of general formula (1) possess asymmetric centers, and therefore geometric and optical isomers are possible. Such compounds may be conveniently prepared from optically active amines of formula (2) and/or from optically active aminoacid intermediates by the methods described above.

The compounds of general formula (1) are basic compounds and may be used as such or pharmaceutically acceptable acid addition salts may be prepared by treatment with various inorganic or organic acids, such as hydrochloric, hydrobromic, sulfuric, phosphoric, acetic, lactic, succinic, fumaric, malic maleic, tartaric, citric, benzoic, methanesulfonic or carbonic acids.

The compounds of general formula (1) possess useful pharmaceutical properties. In particular they possess useful sedative and antiepileptic properties. These activites were assessed by standard methods. Antiepileptic activity was measured by assessing a compound's ability to prevent the hind limb tonic extension component of the seizure in groups of mice induced by maximal electroshock after oral or intraperitoneal administration according to the procedures of the Epilepsy Branch, NINCDS as published by R. J. Porter, et al., Cleve. Clin. Quarterly 1984, 51. 293, and compared to the standard agents dilantin and phenobarbital. Activities in the range of 10-400 m/k after oral administration in this assay system were obtained. Sedative activity was assessed by behavioral observation in groups of mice by standard literature procedures. Selected compounds exhibited activity in the range of 30-600 m/k in this assay.

The following non-limiting illustrations and examples are provided to exemplify the preparation of the intermediate amines of formula (2) and their conversion to the novel compounds of general formula (1).

## Preparation of Intermediates

### Illustration 1

### Preparation of 1,2-Diphenyl-2-propylamine hydrochloride

This compound was prepared by suitable modification of the procedures described by Christol, Bull. Soc. Chim. Fr., 1963, 4, 877, and Ho and Smith, Tetrahedron, 1970, 26, 4277 as follows. To a suspension of sodium cyanide (34.3 g, 0.7 mol) in 500 ml of glacial acetic acid and 100 ml of n-butylether at 0°C was added portionwise 200 ml of concentrated sulfuric acid. The ice bath was removed and a solution of 1,2-diphenyl-2-propanol (106 g, 0.5 ml) in 100 ml of n-butylether was added dropwise over a period of 2 hours, then the mixture stirred for 48 hours. The mixture was poured into 1000 cc of ice, and extracted with chloroform. The extracts were washed with water, dried and evaporated to a solid residue which was stirred with hexane (500 ml), filtered and dried to give 85.35 g (72% yield) of N-formyl-1,2-diphenyl-2-propylamine, mp 97-99°C. This was suspended in 1 L of 10% HCl and heated to reflux for 2.5 hours. After cooling in air for 1 hour then in an ice bath for 30 minutes, the white solid which had crystallized was collected by filtration and vacuum dried to give 85.9 g (97% yield) of 1,2-diphenyl-2-propylamine hydrochloride, mp 175-178°C.

### Illustration 2

### Preparation of N-Methyl-1,2-diphenylethylamine

To a stirred two phase solution of 1,2-diphenylethylamine (30.0 g, 0.15 mol) in 300 ml of methylene chloride and 500 ml of water was added sodium carbonate (23.9 g, 0.225 mol) and the solution was cooled to 10°C under nitrogen. Ethyl chloroformate (21.5 ml, 0.225 mol) was added dropwise over a 1 hour period. The reaction was warmed to ambient temperature and stirred at that temperature for 3 hours. The phases were separated and the aqueous phase was extracted with methylene chloride (75 ml). The combined methylene chloride extracts were washed with 1N HCl (200 ml), dried and evaporated to a white solid, 40.3 g. Recrystallization from cyclohexane gave N-carboethoxy-1,2-diphenylethylamine, mp 74-75°C.

To a stirred suspension of lithium aluminum hydride (12.4 g, 0.32 mol) in 300 ml of tetrahydrofuran at 0°C under nitrogen was added dropwise a solution of N-carboethoxy-1,2-diphenylethylamine (35.0 g, 0.13 mol) in 200 ml of tetrahydrofuran. The mixture was heated to reflux for 8 hours. The mixture was cooled in an ice-water bath and water (30 ml), 15% NaOH (13 ml) and water (39 ml) were carefully added to the

mixture. The mixture was warmed to ambient temperature and the precipitated salts were removed by filtration through celite. Removal of solvent gave N-methyl-1,2-diphenylethylamine, 26,8 g as a colorless oil.

Treatment of this oil with maleic acid in ethyl acetate and methanol gave N-methyl-1,2-diphenylethylamine maleate, mp 129-131°C.

Illustration 3

Preparation of N-methyl-1,2-diphenyl-2-propylamine hydrochloride

N-formyl-1,2-diphenyl-2-propylamine (23.6 g, 0.1 mol) was added to a stirred suspension of $LiAlH_4$ (15.0 g, 0.395 mol) in 1 L of dry tetrahydrofuran. After 2 hours the mixture was heated at 35°C for 22 hours, then refluxed for 2 hours, and allowed to cool to room temperature. Water was added to decompose the excess $LiAlH_4$, and the mixture filtered to remove solid salts. Evaporation of the solvent gave 23.0 g of the crude product as a yellow oil. This was dissolved in 180 ml of ethyl acetate and 20 ml of isopropanol and acidified with HCl gas. Upon standing a white solid crystallized which was collected by filtration and vacuum dried at 65°C to give 21.7 g (84%) of N-methyl-1,2-diphenyl-2-propylamine hydrochloride; mp 200-201°C.

Illustration 4

Preparation of (-)-1,2-Diphenyl-2-propylamine

Racemic 1,2-diphenyl-2-propylamine (86 g, 0.4 mol) was dissolved in 0.5 L 95% ethanol, heated to near reflux and added to a solution of (-)-dibenzoyltartaric acid monohydrate (151.9 g, 0.4 mol) in 0.5 L 95% ethanol also at reflux. A white solid crystallized immediately. The mixture was refluxed for 5 minutes, then allowed to cool to ambient temperature. The solid was collected by filtration and dried to give 86.2 g $[\alpha]_D$ = 94.2°, C = 0.5, $CH_3OH$). The filtrate was saved. The solid was suspended in 0.9 L of 95% ethanol, stirred and heated to reflux for 1 hour, allowed to cool to ambient temperature and the white solid collected by filtration and vacuum dried at 80°C for 8 hours to give 60.2 g of (-)-1,2-diphenyl-2-propylamine-(-)dibenzoyl tartrate, mp 194-195°C; $[\alpha]_D$ = -96.0° (C = 0.5, $CH_3OH$). 5.0 g of this salt was dissolved in 250 ml $CHCl_3$ and 200 ml 5% $NH_4OH$ shaken vigorously, the layers separated and the organic phase washed with 3 x 200 ml 5% $NH_4OH$, 2x 200 ml $H_2O$ and dried over $MgSO_4$. The solvent was evaporated to give 1.75 g of (-)-1,2-diphenyl-2-propylamine as an oil. The maleate salt was prepared by dissolving this oil in 25 ml of ethylacetate and adding the solution to a hot solution of maleic acid (1.02 g, 8.87 mmol) in 50 ml of 3/1 ethylacetate/isopropanol. Upon cooling a white solid crystallized, which was collected by filtration and vacuum dried to give 2.05 g of (-)-1,2-diphenyl-2-propylamine maleate, mp 176-177°C, $[\alpha]_D$ = 27.4°, (C = 1, $CH_3OH$).

Illustration 5

Preparation of (+)-1,2-Diphenyl-2-propylamine

The filtrate residue which was saved in Illustration 4, was treated with 1 L $CHCl_3$ and 0.9 L 5% $NH_4OH$, shaken vigorously, the layers separated and the organic phase washed with 4 x 800 ml 5% $NH_4OH$ and 2 x 500 ml $H_2O$, then dried over $MgSO_4$ and evaporated to an oil 32.3 g, which is enriched in (+)-1,2-diphenyl-2-propylamine. This oil (32.3 g, 0.153 mol) was dissolved in 200 ml hot 95% ethanol and added to a stirred solution of (+)-dibenzoyl tartaric acid monohydrate (57.55 g, 0.153 mol) in 600 ml of refluxing 95% ethanol. A white solid crystallized immediately, which was stirred at reflux for 5 minutes, then allowed to cool to ambient temperature. The solid was collected by filtration and vacuum dried at 80°C for 8 hours to give 71.6 g of (+)-1,2-diphenyl-2-propylamine (+)-dibenzoyltartrate, mp 197-198°C, $[\alpha]_D$ = +95.8°, (C = 0.5, $CH_3OH$). 5.0 g of this salt was dissolved in 250 ml $CHCl_3$ and 200 ml 5% $NH_4OH$, shaken vigorously, the layers separated and the organic phase washed with 3 x 200 ml 5% $NH_4OH$ and 2 x 200 ml $H_2O$ dried over $MgSO_4$. The solvent was evaporated to give 1.75 g of (+)-1,2-diphenyl-2-propylamine as an oil. The

maleate salt was prepared by dissolving this oil in 25 ml ethyl acetate and adding the solution to a hot solution of maleic acid (1.02 g, 8.78 mmol) in 50 ml 3/1 ethyl/acetate/isopropanol. Upon cooling a white solid crystallized, which was collected by filtration and vacuum dried to give 2.06 g of (+)-1,2-diphenyl-2-propylamine maleate, mp 177-178°C, $[\alpha]_D$ = +27.3° (C = 1, $CH_3OH$).

Example 1

Preparation of N-(1,2-diphenyl-1-methylethyl)-2S-pyrrolidinecarboxamide

To a stirred solution of 1,2-diphenyl-2-propylamine (0.085 mol) in 500 ml of chloroform under nitrogen was added N-CBZ-L-proline (0.11 mol), and then a solution of dicyclohexylcarbodiimide (0.1 mol) in 100 ml of chloroform and the mixture stirred for 14 hours. The precipitated solid was removed by filtration and the solvent evaporated. The residue was dissolved in 500 ml of methylene chloride, filtered and evaporated to a yellow oil. This was treated with ether (750 ml) and 500 ml of ice cold water, basified with 5 ml of 50% NaOH, the layers shaken and separated. The ether layer was washed with water (2 x 125 ml), dried and evaporated to an oil. This was dissolved in 500 ml of methanol and 50 ml of 10% HCl, and hydrogenated at 40 psi in a Parr apparatus over 3.0 g of 10% Pd/C catalyst for 4 hours. The catalyst was removed by filtration, and the solvent evaporated to a white solid. This was dissolved in 80 ml of hot methanol and treated with 200 ml of ether. Upon cooling a solid crystalized which was recrystallized from 100 ml of isopropanol and 100 ml of methanol to give of N-(1,2-diphenyl-1-methylethyl)-2S-pyrrolidinecarboxamide hydrochloride, which after vacuum drying at 80 °C for 24 hours had mp 99-102°C.

Example 2

Preparation of N-(1,2-diphenyl-1-methylethyl)-2R-pyrrolidinecarboxamide

By procedures essentially the same as those described in Example 1 and by substituting N-CBZ-D-proline for N-CBZ-L-proline; the corresponding N-(1,2-diphenyl-1-methylethyl)-2R-pyrrolidinecarboxamide, mp 91-95°C, is prepared.

Example 3

Preparation of N-(1,2-diphenyl-1-methylethyl)-2-piperidinecarboxamide hydrochloride

By procedures essentially the same as those described in Example 1 and by substituting N-CBZ-pipecolinic acid for N-CBZ-L-proline; the corresponding N-(1,2-diphenyl-1-methylethyl)-2-piperidinecarboxamide hydrochloride, mp 233-237°C, is prepared.

Example 4

Preparation of N-(1,2-diphenyl-1-methylethyl)-L-thiazolidine-4-carboxaimide hydrochloride

To a stirred solution of 1,2-diphenyl-2-propylamine (14.94 g, 0.071 mol) and BOC-L-thiazolidine-4-carboxylic acid (16.5 g, 0.071 mol) in 350 ml of chloroform was added dicyclohexylcarbodiimide (14.61 g, 0.071 mol) and the mixture stirred for 16 hours, filtered and evaporated to an oily residue. This was dissolved in ethylacetate (200 ml), filtered and an additional 200 ml of ethylacetate added. The solution was washed with 5% cold HCl (2 x 200 ml), dried and evaporated to a pale yellow oil, 26.4 g. This was dissolved in 200 ml ethylacetate and acidified with HCl gas. Upon standing a solid was obtained which was recrystallized from 200 ml of ethanol then 200 ml of 1:1 isopropanol:ethanol containing a trace of water to give after drying N-(1,2-diphenyl-1-methylethyl)-L-thiazolidine-4-carboxamide hydrochloride as a white solid, mp 229-230°C.

Example 5

Preparation of N-(1,2-Diphenylethyl)-2S-pyrrolidinecarboxamide

By procedures essentially the same as those described in Example 1 and by substituting 1,2-diphenylethylamine for 1,2-diphenyl-2-propylamine; the corresponding N-(1,2-diphenylethyl)-2S-pyrrolidinecarboxamide may be prepared.

Example 6

Preparation of N-Methyl-N-(1,2-diphenylethyl)-2S-pyrrolidinecarboxamide

By procedures essentially the same as those described in Example 1 and by substituting N-methyl-1,2-diphenyl-ethylamine for 1,2-diphenyl-2-propylamine; the corresponding N-methyl-(1,2-diphenylethyl)-2S-pyrrolidine-carboxamide may be prepared.

Example 7

Preparation of N-Methyl-N-(1,2-diphenyl-1-methylethyl)-2S-pyrrolidinecarboxamide

By procedures essentially the same as those described in Example 1 and by substituting N-methyl-1,2-diphenyl-2-propylamine for 1,2-diphenyl-2-propylamine; the corresponding N-methyl-N-(1,2-diphenyl-1-methylethyl)-2S-pyrrolidinecarboxamide may be prepared.

Example 8

Preparation of N-(1R-1,2-Diphenyl-1-methylethyl)-2S-pyrrolidinecarboxamide and N-(1S-1,2-diphenyl-1-methylethyl)-2S-pyrrolidinecarboxamide

By procedures essentially the same as those described in Example 1 and by substituting (+)-1,2-diphenyl-2-propylamine or (-) 1,2-diphenyl-2-propylamine for (±)-1,2-diphenyl-2-propylamine either the corresponding N-(1R-1,2-diphenyl-1-methylethyl)-2S-pyrrolidinecarboxamide or N-(1S-1,2-diphenyl-1-methylethyl)-2S-pyrrolidinecarboxamide may be prepared essentially free of enantiomeric and diastereoisomeric forms.

## Claims

1. A compound of the formula

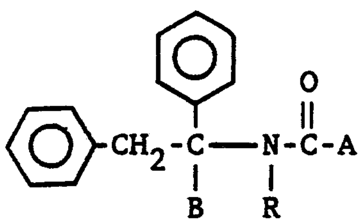

wherein A is 2-pyrrolidinyl, 2-piperidinyl or 4-thiazolidinyl and R and B are independently selected from hydrogen and methyl.

2. N-(1,2-Diphenyl-1-methylethyl)-2S-pyrrolidine-carboxamide.

3. N-(1,2-Diphenyl-1-methylethyl)-2R-pyrrolidine-carboxamide.

4. N-(1,2-Diphenyl-1-methylethyl)-2-piperidine-carboxamide hydrochloride.

5. N-(1,2-Diphenyl-1-methylethyl)-L-thiazolidine-4-carboxamide hydrochloride.

Claims for the following contracting state: ES

1. A process for preparing a compound of the formula:

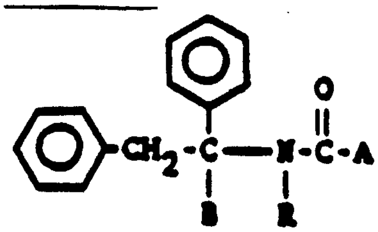

(1)

wherein A is 2-pyrrolidinyl, 2-piperidinyl or 4-thiazolidinyl and R and B are independently selected from hydrogen or methyl, which comprises reacting an amino of the formula

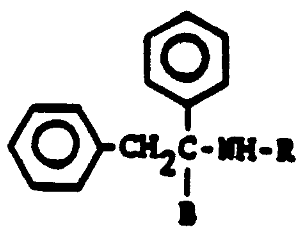

with a suitably N-protected cyclic amino acid, where the nitrogen is protected as a urethane and thereafter removing the protecting group by catalytic hydrogenation or treatment with an acid.

2. A process for preparing a compound according to claim 1 wherein the compound is N-(1,2-diphenyl-1-methylethyl) 2S-pyrrolidinecarboxamide.

3. A process for preparing a compound according to claim 1 wherein the compound is N-(1,2-diphenyl-1-methylethyl)-2R-pyrrolidinecarboxamide.

4. A process for preparing a compound according to claim 1 wherein the compound is N-(1,2-diphenyl-1-methylethyl)-2-piperidinecarboxamide hydrochloride.

5. A process for preparing a compound according to claim 1 wherein the compound is N-(1,2-diphenyl-1-methylethyl)-L-thiazolidine-4-carboxamide hydrochloride.